# FASCICULE DE BREVET EUROPEEN

(11) **EP 1 446 055 B1**
(45) Date de publication et mention de la délivrance du brevet: **25.02.2009**
(21) Numéro de dépôt: 02795374.4
(22) Date de dépôt: 14.11.2002
(51) Int. Cl.: A61B 17/04

(54) **PASSE-FIL CHIRURGICAL**
CHIRURGISCHER NÄHFADENFÜHRUNGSKÖRPER
SURGICAL SUTURE GUIDE

(30) Priorité: 14.11.2001 FR 0114727
(43) Date de publication de la demande: 18.08.2004
(73) Titulaire: Dubernard, Pierre, 69006 Lyon (FR)
(72) Inventeur: Dubernard, Pierre, 69006 Lyon (FR)
(74) Mandataire: Martin, Didier Roland Valéry
(86) Numéro de dépôt international: PCT/FR2002/003894
(87) Numéro de publication internationale: WO 2003/041592

(56) Documents cités:
- WO-A-01/80747
- WO-A-93/13714
- US-A- 2 897 820
- US-A- 4 493 323
- US-A- 5 037 433
- US-A- 5 387 221
- US-A- 5 391 174
- "Catalogue de la Maison Drapier et fils : instruments de chirurgie, orthopédie - prothèse - bandages" 1912, MAISON DRAPIER ET FILS , PARIS Extrait de l'Internet: URL:http://web2.bium.univ-paris5.fr/livanc /?cote=25513&do=chapitre> * pages 10,14,21 *

## Description

### DOMAINE TECHNIQUE

La présente invention se rapporte au domaine technique des instruments chirurgicaux servant à assurer le passage de fils chirurgicaux, au bout desquels des aiguilles chirurgicales sont montées, à travers des organes creux du corps de mammifères et en particulier de l'homme, en vue de réaliser une anastomose entre ledit organe creux et un autre organe du corps humain.

La présente invention concerne un passe-fil chirurgical destiné à assurer le passage d'un ou plusieurs fils chirurgicaux associés chacun respectivement à une aiguille chirurgicale à travers un organe creux du corps humain, en vue de réaliser une anastomose entre ledit organe creux et un autre conduit ou organe du corps humain.

### TECHNIQUE ANTERIEURE

De manière classique, il est déjà connu dans le domaine chirurgical d'avoir recours à des porte-aiguilles se présentant généralement sous la forme de pinces chirurgicales classiques, qui permettent, par pincement direct de l'aiguille, de diriger l'aiguille et le fils vers et dans la zone où sera réalisée l'anastomose.

De tels porte-aiguilles sont largement connus et répandus et peuvent être utilisés avec plus ou moins d'efficacité et de facilité lors d'opérations chirurgicales par voie ouverte ou coelioscopique.

Les porte-aiguilles classiques ne sont néanmoins pas facilement utilisables de manière générale dans tous les divers types d'opérations chirurgicales, et leur utilisation demeure délicate, en particulier lorsque l'on souhaite pratiquer une anastomose impliquant au moins un organe creux à travers lequel le fil et l'aiguille doivent transiter.

Ainsi, à titre d'exemple, dans le cas d'une prostatectomie radicale, il s'avère délicat et difficile d'avoir recours à des porte-aiguilles classiques pour réaliser une anastomose entre la vessie et l'urèthre, par exemple par voie coelioscopique.

En effet, ce type d'opération chirurgicale, lorsqu'elle est effectuée par voie coelioscopique, ne permet pas au chirurgien d'avoir une excellente vision à travers l'urèthre et la vessie pour la mise en place et la réalisation des points de suture uréthro-vésicaux. En effet, la vision à travers la caméra chirurgicale s'effectue en deux dimensions (2D), ce qui ne correspond pas à une vision optimale de la zone d'opération.

Par ailleurs, l'opération de passage des fils et de l'aiguille s'effectue au fond du pelvis et à travers un trocart de passage dans une zone de volume extrêmement réduit, ce qui en définitive rend l'opération extrêmement délicate pour le chirurgien à l'aide de porte-aiguilles classiques.

De plus, en raison des conditions difficiles d'opération, le temps total nécessaire à l'anastomose est relativement long, alors que l'on cherche maintenant précisément à réduire autant que possible la durée des opérations chirurgicales pour en réduire les contraintes pour le patient.

On connaît par ailleurs du document WO-93/13714-A2 un passe-fil conforme au préambule de la revendication 1 annexée.

### EXPOSE DE L'INVENTION

L'objet de l'invention vise en conséquence à porter remède aux différents inconvénients énumérés précédemment et à proposer un nouveau passe-fil chirurgical permettant de faciliter grandement le passage des fils chirurgicaux à travers les corps creux, et en particulier de l'urèthre, et d'améliorer la réalisation des sutures en cas d'anastomose, tout en réduisant la durée totale des interventions chirurgicales.

Un autre objet de l'invention vise à proposer un nouveau passe-fil chirurgical permettant de faciliter la mise en place des points de suture en cas d'anastomose.

Un-autre objet de l'invention vise à proposer un nouveau passe-fil chirurgical permettant au chirurgien une plus grande facilité d'intervention et une meilleure adaptabilité à différentes situations chirurgicales.

Un autre objet de l'invention vise à proposer un nouveau passe-fil chirurgical permettant, à l'aide de moyens simples, d'assurer la commande du déplacement des fils et aiguilles chirurgicales à travers un corps creux.

Un autre objet de l'invention vise à proposer un nouveau passe-fil chirurgical permettant au chirurgien de réduire fortement la durée d'intervention tout en améliorant la précision de la mise en place des points de suture nécessaires à l'anastomose.

Un objet additionnel de l'invention vise à proposer un nouveau passe-fil chirurgical particulièrement adapté à la réalisation d'une anastomose uréthro-vésicale par voie rétro-pubienne.

Les objets assignés à l'invention sont atteints à l'aide d'un passe-fil chirurgical caractérisé en ce qu'il comprend :
- un tube passe-fil pourvu intérieurement et axialement d'au moins un canal, avec une entrée et une sortie situées respectivement vers les parties distales et proximales dudit tube, pour le passage d'un fil associé à une aiguille, la partie proximale du tube passe-fil comportant une portion terminale courbe définissant une partie convexe et une partie concave,
- un moyen de poussée destiné à être enfilé axialement dans le canal par son entrée, pour y coulisser et assurer la poussée de l'aiguille et
- son extériorisation par la sortie, ledit moyen de poussée comportant une tête de commande accessible et hors dudit tube,
caractérisé en ce que la sortie du canal débouche latéralement dans la partie convexe ou dans la partie concave, vers la partie proximale du tube passe-fil, l'axe de sortie du canal étant angulairement différent de la direction générale de ladite partie proximale.

### DESCRIPTIF SOMMAIRE DES DESSINS

D'autres avantages et objets de l'invention seront explicités plus en détails à la lecture de la description qui suit, et à l'aide des dessins annexés fournis à titre purement explicatif et non limitatif, dans lesquels :
- La figure 1 représente, selon une vue schématique en coupe transversale longitudinale, un premier exemple de passe-fil chirurgical
- La figure 2 représente, selon une vue schématique latérale, un second exemple de réalisation d'un passe-fil chirurgical.
- La figure 3 représente, selon une vue en coupe transversale, un détail de réalisation de la section du tube passe-fil selon le premier exemple de réalisation.
- La figure 4 représente, selon une vue en coupe transversale effectuée selon la ligne IV-IV de la figure 2, un détail de réalisation de la section d'un tube passe-fil conforme à l'invention correspondant à une seconde variante de réalisation.
- La figure 5 représente, selon une vue latérale, un détail de réalisation de la partie proximale du tube passe-fil conforme à l'invention correspondant à une autre variante de réalisation.
- Les figures 6 et 7 représentent des détails de réalisation de formes diverses de parties proximales de tubes passe-fils.
- La figure 8 montre, selon une vue en coupe partielle, un détail de réalisation d'une variante préférée de l'invention.
- La figure 9 représente, de manière schématique, les principes de mise en oeuvre du tube passe-fil selon l'invention dans le cas d'une anastomose uréthro-vésicale.

### MEILLEURE MANIERE DE REALISER L'INVENTION

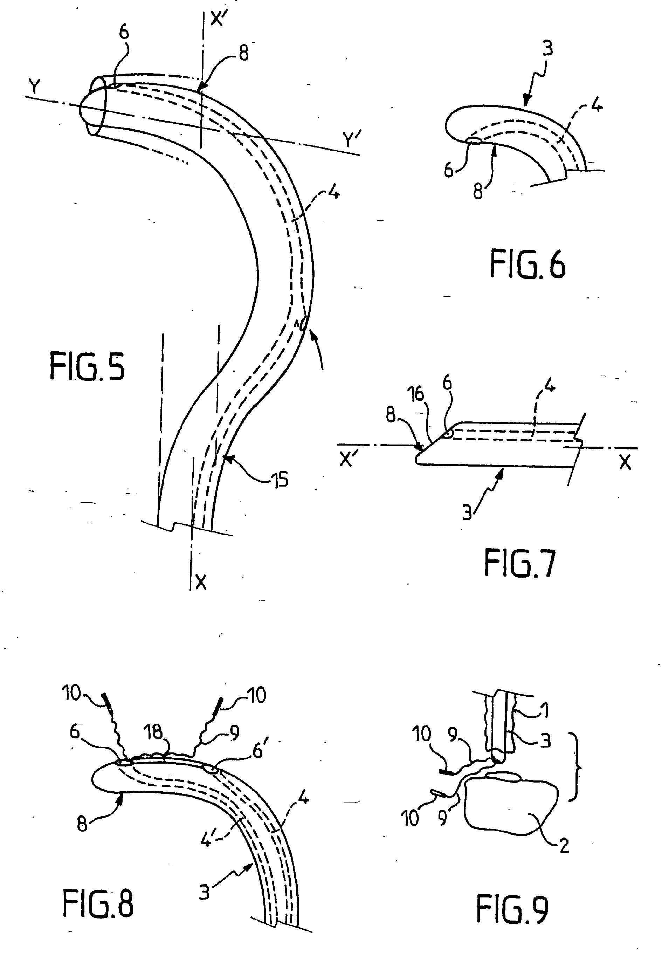

Dans la description qui suit, il sera plus particulièrement fait référence à la réalisation d'un tube passe-fil chirurgical particulièrement adapté à la réalisation d'une anastomose uréthro-vésicale consistant à raccorder, par suturation, l'urèthre 1 à la vessie 2 (figure 9).

Néanmoins, au sens de l'invention, le tube passe-fil chirurgical conforme à l'invention pourrait être utilisé et appliqué dans d'autres types d'opérations chirurgicales, du type anastomose, nécessitant le passage de fils chirurgicaux et d'aiguilles à l'intérieur de corps creux, sans que l'invention puisse être considérée comme étant limitée à la stricte application à une anastomose uréthro-vésicale.

L'instrument chirurgical conforme à l'invention est formé par un passe-fil chirurgical qui comprend un tube passe-fil 3, de forme généralement tubulaire ou pseudo-tubulaire, formant une gaine réalisée par exemple à partir d'inox ou de titane ou d'autres matériaux métalliques, ou encore à partir de matériaux plastiques souples ou semi-rigides. Dans tous les cas, le matériau utilisé sera compatible avec toute utilisation chirurgicale et répondra aux normes et contraintes requises dans le domaine.

Le tube passe-fil 3, par exemple d'un diamètre moyen de l'ordre de 8 millimètres dans le cas d'une application à une anastomose uréthro-vésicale, est pourvu intérieurement et axialement d'au moins un canal 4 avec une entrée 5 et une sortie 6 situées respectivement vers ou au voisinage des parties distale 7 et proximale 8 dudit tube, pour permettre le passage d'un fil chirurgical 9 associé et relié par son extrémité à une aiguille 10.

Les aiguilles 10 pourront être de tout type classique, telles que celles utilisées en matière chirurgicale, et être par exemple droites, ou légèrement recourbées à l'extrémité ou encore béquillées. Les fils 9 seront de tout type classique, tels que ceux utilisés classiquement en chirurgie et seront avantageusement résorbables.

Le passe-fil chirurgical conforme à l'invention comprend également un moyen de poussée 11 destiné à être enfilé axialement dans le canal 4 par son entrée 5, pour y coulisser et assurer la poussée de l'aiguille 10 et son extériorisation par la sortie 6, ledit moyen de poussée 11 comportant une tête de commande 12 accessible extérieurement au tube passe-fil 3, c'est-à-dire située hors dudit tube.

Grâce à la structure de cet instrument chirurgical, le tube passe-fil 3, qui correspond de manière générale à un beniqué, permet, après introduction dudit passe-fil dans l'urèthre 1, de faire transiter de manière extrêmement simple et rapide l'aiguille 10 dans le canal 4 grâce à la poussée effectuée directement ou indirectement sur l'aiguille 10 par le moyen de poussée 11 qui est actionné par le chirurgien.

Avantageusement, le moyen de poussée 11 sera constitué par une tige réalisée en matériau plastique ou métallique, de rigidité et de souplesse suffisante pour assurer la poussée mécanique nécessaire ou indirecte de l'aiguille 10 tout en s'adaptant au profil longitudinal droit ou courbe du canal 4.

Avantageusement, et selon une autre variante, le moyen de poussée 11 pourra être réalisé sous la forme d'un curseur (non représenté) solidaire de l'aiguille 10 et accessible à l'utilisateur par l'intermédiaire d'une tête de commande faisant saillie hors d'une fente (non représentée) ménagée à travers le canal 4.

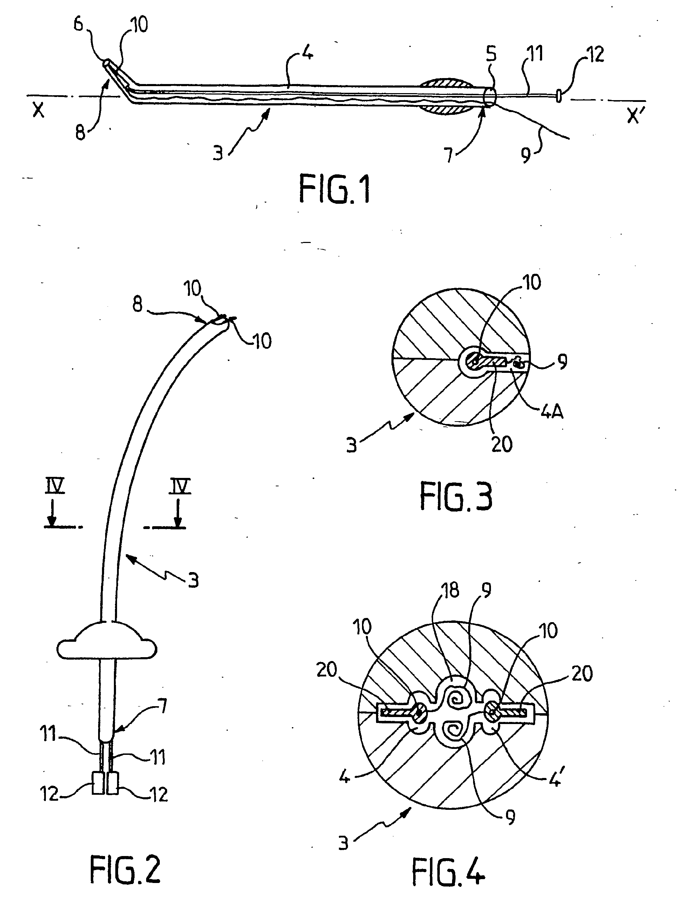

La tête de commande 12 sera formée par tous moyens classiques permettant une bonne préhension du moyen de poussée ainsi qu'une éventuelle rotation, pour être formée, tel qu'illustré par exemple à la figure 2, d'anses facilement préhensibles manuellement.

Avantageusement, et pour favoriser la sortie de l'aiguille du canal 4 selon une direction propice à une bonne mise en place des points de suture par le chirurgien, le canal 4 débouchera sensiblement radialement ou latéralement vers la partie proximale 8 du tube passe-fil 3 et à travers la paroi du tube 3, en considération de la direction axiale Y-Y'de la partie proximale 8.

Dans ce dernier cas (figure 5), le canal 4 débouchera sensiblement perpendiculairement au plan de sortie du tube 3.

Dans tous les cas, l'axe de sortie du canal 4 est angulairement différent de la direction générale du tube ou de sa partie proximale 8.

Ainsi, selon l'invention, le passe-fil chirurgical est caractérisé en ce que la sortie 6 du canal 4 débouche dans - la partie convexe ou concave sensiblement radialement vers la partie proximale 8 du tube passe-fil 3, en considération de la direction axiale principale de la partie proximale 8, la sortie 6 débouchant hors du tube au niveau de la partie proximale 8.

Le tube passe-fil 3 pourra présenter toute forme géométrique appropriée combinant des portions droites ou courbes.

En particulier, et avantageusement tel qu'illustré aux figures, la partie proximale 8 comporte une portion terminale courbe, ceci afin de faciliter la manipulation par le chirurgien lors du passage dans l'urèthre 1, tout en maîtrisant au mieux la zone géométrique d'extériorisation de l'aiguille 10 au voisinage de la zone de suture.

De manière avantageuse, la partie terminale courbe de la partie proximale 8 sera précédée, en direction de la partie distale 7 du tube, d'une portion infléchie 15 (figure 5) pour former de manière générale une partie proximale 8 en forme de béquille en vue d'améliorer la facilité de manipulation.

Tel qu'illustré aux figures 5 et 6, la sortie 6 du canal 4 débouche dans la partie convexe (figure 5) ou concave (figure 6) définie par la partie proximale courbe 8.

Selon une autre forme de réalisation, tel qu'illustré à la figure 7, la partie proximale 8 du tube passe-fil 3 présente une face terminale biseautée 16, dans laquelle débouche la sortie 6 du canal 4.

Tel qu'illustré à la figure 3, le tube passe-fil selon l'invention comporte avantageusement un canal 4 débouchant latéralement sur tout ou partie de la longueur du tube pour le passage du fil 9. Le canal 4 présente également une section transversale formée par deux conduits parallèles et adjacents, 4A et 4B, respectivement, pour le passage du fil 9 et pour le passage de l'aiguille 10 et du moyen de poussée 11. On améliore ainsi grandement la capacité et la facilité de glissement et coulissement de l'aiguille 10 et du fil 9 associé.

Selon une variante particulièrement avantageuse de l'invention et tel qu'illustré aux figures 2, 4 et 8, le tube passe-fil 3 selon l'invention est pourvu de deux canaux 4, 4', sensiblement parallèles et distants l'un de l'autre pour le passage, respectivement chacun d'une aiguille 10, les deux canaux étant reliés entre eux sur toute leur longueur par une gorge centrale longitudinale 18 et axiale destinée au passage du fil 9, le fil étant unique et relié à chacune de ses extrémités aux aiguilles 10, de telle sorte que les deux sorties 6, 6' de chacun des canaux sont reliées par ladite gorge.

Grâce à cette particularité de conception, deux aiguilles peuvent être simultanément introduites dans chacun des canaux, et extériorisées par les sorties 6, 6', tout en étant toujours reliées par un seul fil 9 (logé dans la gorge 18 et s'y déroulant), ce qui facilite grandement l'opération de suture périphérique et permet un gain de temps appréciable de l'opération.

A titre de variante, il est bien évidemment possible de réaliser plus de deux canaux dans le tube passe-fil 3, de manière à permettre le passage de plus de deux aiguilles 10, sans pour autant sortir du cadre de l'invention.

Tel qu'illustré par exemple à la figure 2, le tube passe-fil conforme à l'invention comportera avantageusement deux moyens de poussée 11 associés respectivement à chacun des canaux 4, 4'.

De manière avantageuse, et tel qu'illustré à la figure 8, les deux sorties 6, 6' correspondant à chaque canal 4, 4', débouchent sensiblement radialement ou latéralement hors du tube et de sa paroi, au niveau de la partie proximale 8, par exemple dans la partie convexe pour former une sortie sensiblement centrale ou proximale 6 et une sortie sensiblement latérale ou distale 6', reliée par la gorge 18 qui forme une fente de sortie à travers la paroi.

A titre de variante de réalisation, non représentée aux figures, il est envisageable de réaliser, au moins la partie proximale 8 du tube 3, de telle façon que ladite partie proximale 8 soit montée avec une possibilité de rotation relative autour d'un axe longitudinal, par rapport au reste du tube. Grâce à un tel montage, on améliore fortement les possibilités de manipulation de l'instrument.

Tel qu'illustré aux figures 3 et 4, l'aiguille 10 sera avantageusement supportée, de manière amovible, par un élément pousse-aiguille 20 adapté à la forme et au profil du canal 4, 4A, 4B, pour faciliter le transit de l'aiguille dans ledit canal. Avantageusement, le pousse-aiguille 20 sera monté de manière solidaire, et amovible ou non, du moyen de poussée 11. Dans ce type de montage, la poussée du moyen de poussée 11 s'effectue donc de manière indirecte sur l'aiguille 10. Cette disposition permet d'obtenir un système rechargeable, l'unité formée par la (les) aiguille(s) 10, le pousse-aiguille 20 et le fil 9 formant l'élément rechargeable et insérable dans le système. La fonction de rechargement est adaptable à toutes les variantes de l'invention, et notamment aux variantes présentant un canal 4 formé par deux conduits 4, 4', le fil 9 et l'aiguille 10 associée étant rechargeables.

En opération, le chirurgien, après avoir assuré la fermeture du col vésical de la vessie, procède à la mise en place des points de suture uréthro-vésicaux en introduisant le tube passe-fil dans l'urèthre, de manière à le faire déboucher à proximité du col vésical. Après avoir préalablement saisi là où les aiguille(s) 10 et les fils 9 correspondant dans le canal ou les canaux 4, 4', le chirurgien peut ensuite, par action sur la tête de commande 12, assurer l'extériorisation de la ou des aiguille(s) 10 hors des sorties 6, 6' au voisinage de la zone de suture. Le col vésical est ensuite suturé à l'urèthre en passant l'aiguille de l'intérieur de l'urèthre vers l'extérieur, le fil 9 se déroulant naturellement hors de canaux 4, 4'.

Le passe-fil chirurgical conforme à l'invention correspond donc, dans sa forme préférée de réalisation, à un passe-fil uréthal qui facilite grandement l'anastomose uréthro-vésicale, tout en réduisant fortement le temps total nécessaire pour l'opération, en raison de l'aide apportée : au passage du fil et de l'aiguille, au positionnement correct des aiguilles relativement à la zone de suture et à l'opération finale de nouage.

L'invention concerne également tout procédé ou méthode chirurgicale impliquant l'utilisation du passe-fil conforme à l'invention.

### POSSIBILITE D'APPLICATION INDUSTRIELLE

L'invention trouve son application industrielle dans la conception et la _ fabrication de passe-fil chirurgical, en particulier de passe-fil uréthral destiné à des interventions chirurgicales, notamment des anastomoses uréthro-vésicales.

## Revendications

1. - Passe-fil chirurgical comprenant :
- un tube passe-fil (3) pourvu intérieurement et axialement d'au moins un canal (4), avec une entrée (5) et une sortie (6) situées respectivement vers les parties distales (7) et proximales (8) dudit tube, pour le passage d'un fil (9) associé à une aiguille (10), la partie proximale (8) du tube passe-fil (3) comportant une portion terminale courbe définissant une partie convexe et une partie concave,
- un moyen de poussée (11) destiné à être enfilé axialement dans le canal (4) par son entrée (5), pour y coulisser et assurer la poussée de l'aiguille (10) et son extériorisation par la sortie (6), ledit moyen de poussée comportant une tête de commande (12) accessible et hors dudit tube,
**caractérisé en ce que** la sortie (6) du canal (4) débouche latéralement dans la partie convexe ou dans la partie concave, vers la partie proximale (8) du tube passe-fil (3), l'axe de sortie du canal (4) étant angulairement différent de la direction générale de ladite partie proximale (8).

2. - Passe-fil selon la revendication 1 **caractérisé en ce que** la partie terminale courbe est précédée, en direction de la partie distale du tube, d'une portion infléchie (15) pour former une partie proximale en forme de béquille.

3. - Passe-fil selon l'une des revendications 1 ou 2 **caractérisé en ce que** le canal (4) présente une section transversale formée par deux conduits (4A, 4B) parallèles et adjacents, l'un (4A) pour le passage du fil (9), l'autre (4B) pour le passage de l'aiguille (10) et du moyen de poussée (11).

4. - Passe-fil selon l'une des revendications 1 à 3 **caractérisé en ce que** le tube passe-fil (3) est pourvu d'un deuxième canal (4'), les deux canaux (4, 4') étant sensiblement parallèles et distants l'un de l'autre pour le passage, respectivement, chacun d'une aiguille(10), les deux canaux (4, 4') étant reliés entre eux sur toute leur longueur par une gorge (18) longitudinale et axiale destinée au passage du fil (9) qui est relié à chacune des aiguilles (10), de telle sorte que les deux sorties (6, 6') de chacun des canaux (4, 4') sont reliées par la gorge (18).

5. - Passe-fil selon la revendication 4 **caractérisé en ce qu'**il comporte deux moyens de poussée (11), respectivement un pour chacun des canaux (4, 4').

6. - Passe-fil selon l'une des revendications 4 ou 5 **caractérisé en ce que** les deux sorties (6, 6') débouchent sensiblement radialement hors du tube (3) au niveau de la partie proximale (8), pour former une sortie (6) proximale et une sortie (6') distale.

7. - Passe-fil selon l'une des revendications 1 à 6 **caractérisé en ce qu'**au moins la partie proximale (8) du tube est montée avec une possibilité de rotation relative par rapport au reste du tube (3).

8. - Passe-fil selon l'une des revendications précédentes **caractérisé en ce que** l'aiguille (10) est supportée de manière amovible par un élément pousse-aiguille (20) solidaire du moyen de poussée (11).

9. - Passe-fil selon l'une des revendications précédentes **caractérisé en ce que** le système est rechargeable, le fil (9) et la (les) aiguille(s) (10) associée(s) étant rechargeables.

10. - Passe-fil selon l'une des revendications précédentes **caractérisé en ce qu'**il est formé par un passe-fil urèthral.

## Claims

1. Surgical suture guide comprising:
- a suture guide tube (3) provided internally and axially with at least one channel (4), with an inlet (5) and an outlet (6) located respectively towards the distal part (7) and proximal part (8) of said tube, for the passage of a suture (9) associated with a needle (10), the proximal part (8) of the suture guide tube (3) comprising a curved end portion defining a convex part and a concave part,
- a thrust means (11) designed to be inserted axially into the channel (4) by way of its inlet (5), so as to slide therein and push the needle (10) and cause the latter to exit through the outlet (6), said thrust means comprising an accessible control head (12) outside said tube,
**characterised in that** the outlet (6) of the channel (4) opens laterally in the convex part or in the concave part, towards the proximal part (8) of the suture guide tube (3), the outlet axis of the channel (4) being angularly different from the general direction of said proximal part (8).

2. Suture guide according to claim 1, **characterised in that** the curved end part is preceded, in the direction of the distal part of the tube, by an inflected portion (15) so as to form a proximal part in the shape of a crutch.

3. Suture guide according to one of claims 1 or 2, **characterised in that** the channel (4) has a cross section formed by two parallel and adjacent ducts (4A, 4B), one (4A) for the passage of the suture (9), the other (4B) for the passage of the needle (10) and the thrust means (11).

4. Suture guide according to one of claims 1 to 3, **characterised in that** the suture guide tube (3) is provided with a second channel (4'), the two channels (4, 4') being substantially parallel to and at a distance from one another for the passage of a needle (10) in each case, the two channels (4, 4') being connected to one another over their entire length by a longitudinal and axial groove (18) designed for the passage of the suture (9) which is connected to each of the needles (10), so that the two outlets (6, 6') of each of the channels (4, 4') are connected by the groove (18).

5. Suture guide according to claim 4, **characterised in that** it comprises two thrust means (11), one for each of the channels (4, 4').

6. Suture guide according to one of claims 4 or 5, **characterised in that** the two outlets (6, 6') open out of the tube (3) substantially radially at the proximal part (8), so as to form a proximal outlet (6) and a distal outlet (6').

7. Suture guide according to one of claims 1 to 6, **characterised in that** at least the proximal part (8) of the tube is mounted with a possibility of rotation relative to the rest of the tube (3).

8. Suture guide according to one of the preceding claims, **characterised in that** the needle (10) is supported in a removable manner by a needle-pushing element (20) secured to the thrust means (11).

9. Suture guide according to one of the preceding claims, **characterised in that** the system is reloadable, the suture (9) and the associated needle(s) (10) being reloadable.

10. Suture guide according to one of the preceding claims, **characterised in that** it is formed by a urethral suture guide.

## Patentansprüche

1. Chirurgischer Nähfadendurchführungskörper, umfassend:
- ein Nähfadendurctiführungskörper-Rohr (3), im Innern und seitlich ausgestattet mit mindestens einem Kanal (4), mit einem Eintritt (5) und einem Austritt (6), die sich auf dem distalen (7) bzw. dem proximalen (8) Teil des Rohrs befinden, für den Durchgang eines Fadens (9), der mit einer Nadel (10) verbunden ist, wobei der proximale Teil (8) des Nähfadendurchführungskörper-Rohrs (3) einen gekrümmten Endteil umfasst, der einen konvexen Teil und einen konkaven Teil definiert,
- ein Schiebemittel (11), das dazu vorgesehen ist, axial in den Kanal (4) durch seinen Eintrilt (5) eingefädelt zu werden, um in diesen zu gleiten und den Schub der Nadel (10) und ihren Ausgang durch den Austritt (6) sicherzustellen, wobei das Schiebemittel einen Steuerkopf (12) umfasst, der zugänglich ist und außerhalb des Rohrs liegt,
**dadurch gekennzeichnet, dass** der Austritt (6) des Kanals (4) seitlich in den konvexen oder in den konkaven Teil in Richtung auf den proximalen Teil (8) des Nähfadendurchführungskörper-Rohrs (3) mündet, wobei die Austrittsachse des Kanals (4) einen anderen Winkel als die allgemeine Richtung des proximalen Teils (8) aufweist.

2. Nähfadendurchführungskörper nach Anspruch 1, **dadurch gekennzeichnet, dass** dem gekrümmten Enteil in Richtung des distalen Teils des Rohrs ein gebogener Abschnitt (15) vorangestellt ist, um einen proximalen Teil in Form eines Ständers zu bilden.

3. Nähfadendurchführungskörper nach einem der Ansprüche 1 oder 2, **dadurch gekennzeichnet, dass** der Kanal (4) einen Querschnitt, aufweist, der durch zwei parallele und nebeneinander liegende Röhren (4A, 4B) gebildet ist, eine (4A) für den Durchlauf des Nähfadens (9), die andere (4B) für den Durchlauf der Nadel (10) und des Schiebemittels (11).

4. Nähfadcndurchführungskörper nach einem der Ansprüche 1 bis 3, **dadurch gekennzeichnet, dass** das Nähfadendurchführungskörper-Rohr (3) einen zweiten Kanal (4') aufweist, wobei die zwei Kanäle (4, 4') im Wesentlichen parallel und voneinander beabstandet sind für den Durchgang jeweils einer Nadel (10), wobei die zwei Kanäle (4, 1') untereinander auf ihrer gesamten Länge durch eine längsgerichtete und axiale Rille (18) verbunden sind, die für den Durchlauf des Fadens (9) bestimmt isL, der miL jeder der Nadeln (10) verbunden ist, sodass die zwei Austritte (6, 6') jedes der Kanäle (4, 4') durch die Rille (18) verbunden sind.

5. Nähfadendurchführungskörper nach Anspruch 4, **dadurch gekennzeichnet, dass** er zwei Schiebemittel (11), jeweils eines für jeden der Kanäle (4, 4'), umfasst.

6. Nähfadendurchführungskörper nach einem der Ansprüche 4 oder 5, **dadurch gekennzeichnet, dass** die zwei Austritte (6, 6') im Wesentlichen radial außerhalb des Rohrs (3) auf dem Niveau des proximalen Teils (8) münden, um einen proximalen Austritt (6) und einen distalen Austritt (6') zu bilden.

7. Nähfadendurchführungskörper nach einem der Ansprüche 1 bis 6, **dadurch gekennzeichnet, dass** mindestens der proximale Teil (8) des Rohrs mit der Möglichkeit einer relativen Drehung hinsichtlich des restlichen Rohrs (3) montiert ist.

8. Nähfadendurchführungskörper nach einem der vorgehenden Ansprüche, **dadurch gekennzeichnet, dass** die Nadel (10) abnehmbar von einem Nadelschubelement (20), das mit dem Schiebemittel (11) fest verbunden isL, gehalten wird.

9. Nähfadendurchführungskörper nach einem der vorhergehenden Ansprüche, **dadurch gekennzeichnet, dass** das System nachfüllbar ist, wobei der Faden (9) und die verbundene(n) Nadel (n) (10) nachfüllbar ist/sind.

10. Nähfadendurchführungskörper nach einem der vorhergehenden Ansprüche, **dadurch gekennzeichnet, dass** er aus einem Harnröhren-Nähtadendurchführungskörper gebildet ist.
